# EUROPEAN PATENT APPLICATION

(11) **EP 4 427 772 A1**
(43) Date of publication of application: **11.09.2024**
(21) Application number: 23160738.3
(22) Date of filing: 08.03.2023
(51) Int. Cl.: A61M 5/20

(54) **ACOUSTIC SIGNAL GENERATING DEVICE**

(71) Applicant: Sensile Medical AG, 4600 Olten (CH)
(72) Inventor: FORSTER, Richard, 92269 Fensterbach (DE); GORSHÖFER, Andreas, 93149 Nittenau (DE); JAKOB, Michael, 93051 Regensburg (DE); PFRANG, Jürgen, 93183 Kallmünz (DE)
(74) Representative: Bardehle Pagenberg Partnerschaft mbB Patentanwälte Rechtsanwälte

(57) **Abstract**

The present disclosure relates to an acoustic signal generating device (1) for a medical injection device (5). The acoustic signal generating device (1) has a base body (10) comprising a first resilient arm (12) comprising a first free end (13); an abutment surface (19), and an impact surface (18). The first resilient arm (12) is configured to be deflected by a first load being applied on the first resilient arm (12) such that the first free end (13) of the first resilient arm (12) is in compressive engagement with the abutment surface (19), whereby when the first load is increased to a second load the first free end (13) is released from compressive engagement with the abutment surface (19) and the first resilient arm (12) hits the impact surface (18), thereby generating an acoustic signal. Moreover, the present disclosure relates to a medical injection device (5) comprising said acoustic signal generating device (1).

## Description

### 1. Technical field

The present disclosure relates to an acoustic signal generating device for a medical injection device and a medical injection device comprising said acoustic signal generating device.

### 2. Prior art

For medical injection devices, such as auto-injectors or injection pens, it is often necessary to provide feedback to a patient as soon as a certain operating state of the medical injection device has been reached and/or left. For example, in the case of an auto-injector that injects an emergency medication, such as adrenaline in the event of an allergic shock, the patient must be informed when the medication has been fully dispensed. Otherwise, it cannot be ensured that the injection has been interrupted, for example due to a technical malfunction. It is understood that this could result in a significant health risk for the patient.

In addition to a visual indication showing the extent to which a substance has been dispensed from the medical injection device, it has been found that acoustic feedback is often also required. The reasons for this are numerous. For example, it must be ensured that feedback is provided to visually impaired persons. In addition, an injection area may be located in such a way that the visual indication is not in the patient's field of vision. Further, it is understood that feedback on the injection state/progress is also necessary in poor lighting conditions.

Various solutions are known from the prior art for providing acoustic feedback in a medical injection device, for example when an injection process has been completed or has begun. However, these solutions have various drawbacks as set out in the following.

First, acoustic signal generating devices for medical injection devices are regularly designed in such a way that they must be installed under pretension, whereby the pretension serves to generate the acoustic signal, when released. Examples therefor are known from US 10,918,811 B2 and WO 2018/082886 A1. However, acoustic signal generating devices which require to be installed under pretension have various disadvantageous effects. The fact that pretensioning must take place first complicates assembly. In addition, it has been shown that pretensioned acoustic signal generating devices can break due to the pretension in the installed state. Furthermore, the pretensioned acoustic signal generating devices can lead to damage in the medical injection device, for example if creep occurs in surrounding polymer material. Even further, acoustic signal generating devices which must be installed under pretension, particularly when made of a polymer material, can completely or partially lose their pretension which causes the acoustic signal to have a reduced volume or even fail to appear.

Second, prior art acoustic signal generating devices, such as those described in WO 2018/082886 A1, often include assemblies with multiple parts, sometimes in particularly complex arrangements. Regularly, these multiple parts have to be arranged and fixed relative to each other. This increases the assembly effort and the assembly time. In addition, the presence of multiple parts increases the risk of incorrect assembly. Furthermore, an increased number of parts also increases the manufacturing effort as more parts must be produced.

Thus, it is an object of the present disclosure to provide an acoustic signal generating device for a medical injection device and a medical injection device comprising said acoustic signal generating device that overcome the aforementioned drawbacks at least partially.

### 3. Summary of the invention

This object is achieved, at least partly, by an acoustic signal generating device for a medical injection device and a medical injection device comprising said acoustic signal generating device, as defined in the independent claims. Further aspects of the present disclosure are defined in the dependent claims.

In particular, the object is achieved by an acoustic signal generating device for a medical injection device. The acoustic signal generating device having a base body comprising a first resilient arm comprising a first free end; an abutment surface, and an impact surface. The first resilient arm is configured to be deflected by a first load being applied on the first resilient arm such that the first free end of the first resilient arm is in compressive engagement with the abutment surface, whereby when the first load is increased to a second load the first free end is released from compressive engagement with the abutment surface and the first resilient arm hits the impact surface, thereby generating an acoustic signal.

It is understood that different configurations exist which allow that the first free end is released from compressive engagement with the abutment surface. Exemplarily, the first resilient arm may be bent when the first load is increased to the second load such that the first free end is retracted from the abutment surface. Moreover, the abutment surface may be inclined such that the first free end slips from the abutment surface when the first load is increased to the second load. Even further exemplarily, the abutment surface may be arranged on a movable element which moves such that the first free end is released from compressive engagement with the abutment surface when the first load is increased to the second load. Furthermore, it is understood that combinations of these configurations are also possible. It is also to be noted that increasing the first load to the second load may directly and/or indirectly lead to the first free end being released from compressive engagement with the abutment surface. Exemplarily, when the first load is increased to the second load an additional element may support releasing the compressive engagement of the first free end and the abutment surface. A particular example for this is provided below.

The acoustic signal generating device may be configured for medical injection devices such as auto-injectors, injection pens, and/or syringes.

Said base body may have a shape which allows the acoustic signal generating device to be arranged and/or retained within the medical injection device. Exemplarily, the base body may be adapted to an inside geometry, such as a recess, of medical injection devices.

The base body may comprise individual parts. Exemplarily, the impact surface may be arranged on a separate part of the base body, wherein this separate part comprises a material which allows for an acoustic signal with a certain pitch and/or volume. However, preferably the base body comprises one part having the impact surface and the abutment surface, wherein to said one part the first resilient arm is attached.

Exemplarily, the first resilient arm may be configured to be tensioned by a force which is applied on the first resilient arm in a direction being substantially perpendicular to a longitudinal direction of the first resilient arm. Further, that the first free end is in compressive engagement with the abutment surface may refer to the aspect that the first free end exerts a pressure onto the abutment surface.

Exemplarily, the term "resilient" as used throughout the present disclosure may refer to the ability of a material to withstand elastic deformation without deforming plastically. Hence, the term "resilient arm" according to the present disclosure may refer to an elongated element which has an attached end being attached to another component and a free end which is free and movable relative to the attached end by elastically deforming the resilient arm. Further, it is understood that the term "resilient arm" may be also described as "spring arm".

The acoustic signal generating device according to the present disclosure has different advantages wherein two of them are described in the following in further detail.

First, the acoustic signal generating device does not need to be installed and/or maintained under pretension for generating an acoustic signal. Hence the assembly of the acoustic signal generating device with a medical injection device is facilitated. Moreover, it can be avoided that the acoustic signal generating device breaks due to pretension in the installed state. Furthermore, the acoustic signal generating device does not lead to damages in medical injection devices due to pretension. Even further, the acoustic signal generating device is not at risk to lose pretension when installed.

Second, compared to prior art acoustic signal generating devices the configuration of the present acoustic signal generating device allows for a reduced number of parts and/or less complex arrangements. Hence, assembly effort and/or assembly time may be reduced. In addition, manufacturing effort and/or the risk of incorrect assembly can be minimized.

It is understood that the above-described advantages may also apply for the following in different expressions.

The base body may further comprise a second resilient arm, wherein the abutment surface is arranged on the second resilient arm, whereby the second resilient arm is configured such that when the first load is increased to the second load the second resilient arm is deflected to an extent that the first free end is released from compressive engagement with the abutment surface. This configuration may allow a lower load to be required to achieve the separation of the first free end and the abutment surface. This is as said separation may be facilitated by the deflection of the second resilient arm. By said lower load being required to achieve the separation a malfunction, such as a blocking of the acoustic signal generating device, can be avoided. Moreover, the base body may further comprise a third resilient arm being configured to be deflected such that when the first load is increased to the second load the third resilient arm disengages the abutment surface from compressive engagement with the first free end. This configuration may allow for a particularly precisely adjusted load which is required for the separation of the first free end and the abutment surface. Exemplarily, the third resilient arm may deflect the first resilient arm and/or the second resilient arm. Particularly, the third resilient arm may deflect the second resilient arm such that the abutment surface is retracted from compressive engagement with the first free end. It is understood that the deflection of the third resilient arm may be directly associated with the deflection of the first resilient arm, e.g., if the arms are kinematically coupled. Alternatively, the deflection of the third resilient arm may be indirectly associated with the deflection of the first resilient arm. Exemplarily, when the first load is increased to the second load, the third resilient arm may be deflected independently of the first resilient arm by means of an intermediate element which applies the force onto the first resilient arm. A respective example is set out in the following paragraph.

Optionally, the first resilient arm, the second resilient arm, and/or the third resilient arm are arranged such that moving a substantially planar actuation surface against the acoustic signal generating device by a first distance presses the first free end against the abutment surface, wherein further moving the substantially planar actuation surface against the acoustic signal generating device by an additional second distance deflects the third resilient arm such that it disengages the abutment surface from engagement with the first free end. It is understood that moving the actuation surface by said first distance against the acoustic signal generating device may be associated with the first load being applied on the first resilient arm, wherein moving the actuation surface by said second distance against the acoustic signal generating device may be associated with the second load being applied on the first resilient arm. Hence, it is understood that the deflection of the third resilient arm may be indirectly associated with the deflection of the first resilient arm. Thus, the skilled person further understands that the first load being increased to the second load may indirectly lead to the first free end being released from compressive engagement with the abutment surface.

Further, it is understood that the acoustic signal generating device may be configured such that when moving said substantially planar actuation surface against the acoustic signal generating device the actuation surface may first contact the third resilient arm or the first resilient arm. Alternatively, the actuation surface may contact the third resilient arm and the first resilient arm at the same time. By said arrangement of the resilient arms a facilitated actuation of the acoustic signal generating device may be achieved. Particularly, a simple actuation element within a respective medical injection device may be utilized to actuate the acoustic signal generating device.

The second resilient arm may comprise a second free end and the third resilient arm may comprise a third free end, wherein the second free end and the third free end are configured to engage with each other when the third resilient arm is deflected. Optionally, the third free end is configured to engage with the first free end and the second free end when the third resilient arm is deflected. Exemplarily, the third free end may be pressed into a gap formed between the first free end and the second free end. Hence, separating the first free end from the second free end may be achieved even more reliable.

The base body may be integrally formed with the first resilient arm, the second resilient arm, the third resilient arm, the abutment surface, and/or the impact surface. Hence, in other words, the base body may be formed as one part together with the first resilient arm, the second resilient arm, the third resilient arm, the abutment surface, and/or the impact surface. The term "integrally formed" may refer to the aspect that no material boundaries can be identified between the respective elements. Exemplarily, when the the base body is integrally formed with the first resilient arm, the second resilient arm, the third resilient arm, the abutment surface, and the impact surface, then no material boundary can be identified between said elements. Preferably, the base body is integrally formed with at least two of the resilient arms. Integrally forming the base body with the first resilient arm, the second resilient arm, the third resilient arm, and/or the impact surface particularly allows for a reduced number of parts and/or less complex arrangements. Hence, as above, assembly effort, assembly time, manufacturing effort, and/or the risk of incorrect assembly may be reduced.

Further, the base body, the first resilient arm, the second resilient arm, the third resilient arm, the abutment surface, and/or the impact surface may be injection moulded. Injection moulding is a highly efficient process which exemplarily allows for high production rates. Hence, the time required for manufacturing the acoustic signal generating device may be reduced. This particularly applies when the base body, the first resilient arm, the second resilient arm, the third resilient arm, and the impact surface are integrally formed and injection moulded. In this case assembly effort, assembly time, manufacturing effort, and/or the risk of incorrect assembly may be reduced significantly compared to prior art acoustic signal generating devices.

Moreover, the first resilient arm, the second resilient arm, and/or the third resilient arm may be arranged such that they return to the initial not deflected state after being unloaded. This can be achieved, for example, by the elastic material behaviour of the resilient arms. Hence, the acoustic signal generating device may be reused, i.e., repeatedly generate an acoustic signal. Particularly, no additional steps may be required to transfer the acoustic signal generating device into a position where reuse is possible. In addition, returning to the initial not tensioned state prevents creep and/or other material fatigue from occurring in the acoustic signal generating device.

Optionally, the first resilient arm, the second resilient arm, and/or the third resilient arm do not contact each other when not being loaded. This can further ensure that the resilient arms are not under tension if no actuation, namely tensioning of the first resilient arm, of the acoustic signal generating device takes place. Hence, the above-described disadvantages which regularly apply for pretensioned acoustic signal generating devices may be avoided.

The acoustic signal may have an acoustic pressure of at least 40 dB, optionally at least 60 dB, further optionally at least 80 dB, and even further optionally at least 100 dB. These values have proven to allow for a sufficient volume of the acoustic signal for different applications. The acoustic pressure which may be achieved by the acoustic signal generating device can be exemplarily influenced by the selected material(s) and/or to which extent the first resilient arm can be tensioned against the second resilient arm.

Optionally, the base body has an elongated hollow shape, wherein the base body optionally has a substantially hollow cylindrical shape. An elongated hollow shape is preferred as it allows for elements such as plungers to be guided through. A substantially hollow cylindrical shape is particularly preferred as this allows the acoustic signal generating device to be installed in several regular medical injection devices while the hollow shape allows for elements such as plungers to be guided through. Hence, summarizing, installation space may be efficiently used by the base body having such shapes.

The first resilient arm and/or the third resilient arm may be movable in a direction which is substantially parallel to the longitudinal direction of the base body. Thus, exemplarily the above-described actuation surface with the mentioned movement may serve to move the first resilient arm and the third resilient arm.

Further, the first resilient arm and/or the third resilient arm may comprise a curved portion that extends substantially along the circumferential direction of the base body. Exemplarily, the first resilient arm and/or the third resilient arm may at least partially extend along the circumference of the base body having a substantially hollow cylindrical shape. Thus, the shape of the resilient arms may be adapted to the shape of the base body. This allows for an overall compact shape of the acoustic signal generating device. Hence, exemplarily in a medical injection device installation space may be utilized more efficient.

The second resilient arm may be substantially movable along the circumferential direction of the base body, wherein optionally the second resilient arm extends substantially parallel to the longitudinal direction of the base body. This configuration is particularly beneficial with the first and/or the third resilient arm being configured as described in the previous two paragraphs. This is as the first and the third resilient arm may be moved towards the second resilient arm along one direction, whereas one linear movement of the second resilient arm may release the first resilient arm. Thus, a simple mechanism may be provided.

Further, the above object is achieved by a medical injection device comprising the acoustic signal generating device as described above. As the medical injection device comprises the acoustic signal generating device as described above, it is understood that the advantages and/or features described regarding the acoustic signal generating device may also apply for the medical injection device.

The medical injection device may further comprise a hollow cylindrical actuation sleeve having an actuation surface for actuating the acoustic signal generating device, wherein the actuation sleeve is arranged slidably relative to the base body, wherein optionally the actuation surface is substantially planar. This configuration allows for the medical injection device comprising the acoustic signal generating device to be configured in a particularly simple manner compared to the prior art solutions.

### 4. Brief description of the accompanying figures

In the following, the accompanying figures are briefly described:
Fig. 1 shows a first acoustic signal generating device according to the present invention in perspective view;
Fig. 2 shows a second acoustic signal generating device according to the present invention in perspective view;
Fig. 3 shows a third acoustic signal generating device according to the present invention in side view;
Fig. 4 shows the third acoustic signal generating device in perspective view;
Fig. 5 shows the third acoustic signal generating device in perspective view together with an actuation sleeve;
Fig. 6 shows the third acoustic signal generating device in a further perspective view;
Figs. 7a-c show the third acoustic signal generating device during actuation;
Fig. 8 shows a medical injection device with the third acoustic signal generating device installed therein, and
Fig. 9 shows a detail of the medical injection device.

### 5. Detailed description of the figures

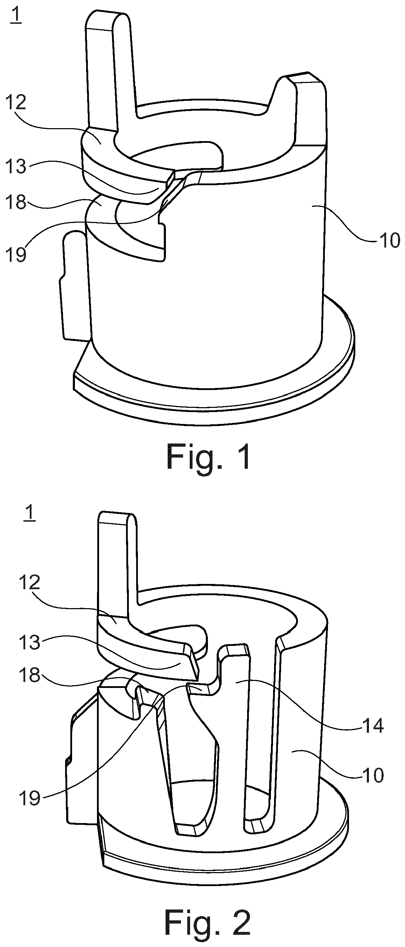

**Fig. 1** shows a first acoustic signal generating device 1 according to the present invention. The acoustic signal generating device 1 has a base body 10 comprising a first resilient arm 12 comprising a first free end 13. Further, the base body 10 comprises an abutment surface 19 and an impact surface 18.

The first resilient arm 12 is configured to be deflected by a first load being applied on the first resilient arm 12 such that the first free end 13 of the first resilient arm 12 is in compressive engagement with the abutment surface 19, whereby when the first load is increased to a second load the first free end 13 is released from compressive engagement with the abutment surface 19 and the first resilient arm 12 hits the impact surface 18, thereby generating an acoustic signal. Particularly, when the first load is increased to the second load the first resilient arm 12 is bent such that the first free 13 end is at least partially retracted from the abutment surface 19 and the abutment surface 19 is furthermore inclined such that a slipping of the first free end 13 from the abutment surface 19 is facilitated.

**Fig. 2** shows a second acoustic signal generating device 1 according to the present invention. The second acoustic signal generating device 1 is configured as the first acoustic signal generating device 1, wherein the base body 10 of the second acoustic signal generating device 1 further comprises a second resilient arm 14.

Said abutment surface 19 is arranged on the second resilient arm 14, whereby the second resilient arm 14 is configured such that when the first load is increased to the second load the second resilient arm 14 is deflected to an extent that the first free end 13 is released from compressive engagement with the abutment surface 19.

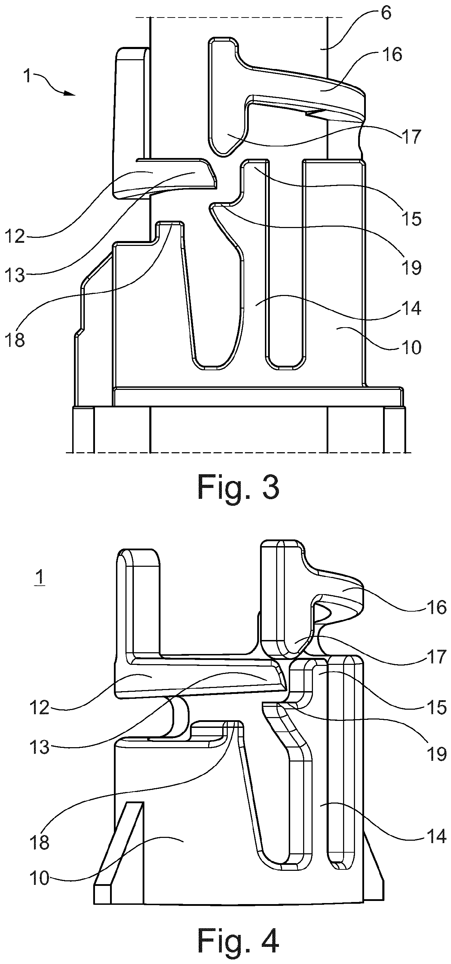

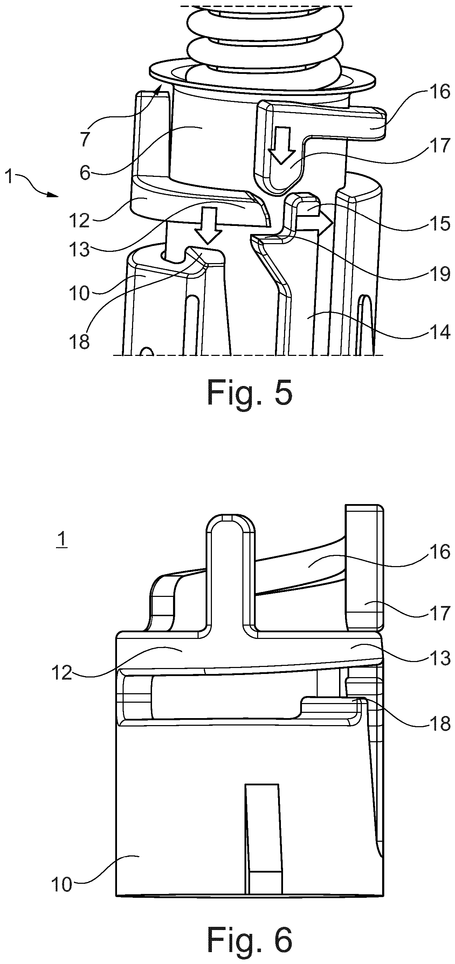

**Figs. 3, 4****,** **5, 6****, and** **7a****-c** show a third acoustic signal generating device 1 according to the present invention. The third acoustic signal generating device 1 is configured as the first and the second acoustic signal generating device 1, wherein the base body 10 of the third acoustic signal generating device 1 further comprises a third resilient arm 16 being configured to be deflected such that it disengages the abutment surface 19 from compressive engagement with the first free end 13. Particularly, the third resilient arm 16 is configured such that when the first load is increased to the second load the third resilient arm 16 disengages the abutment surface 19 from compressive engagement with the first free end 13. Particularly, the second resilient arm 14 comprises a second free end 15 and the third resilient arm 16 comprises a third free end 17, wherein the second free end 15 and the third free end 17 are configured to engage with each other when the third resilient arm 16 is deflected.

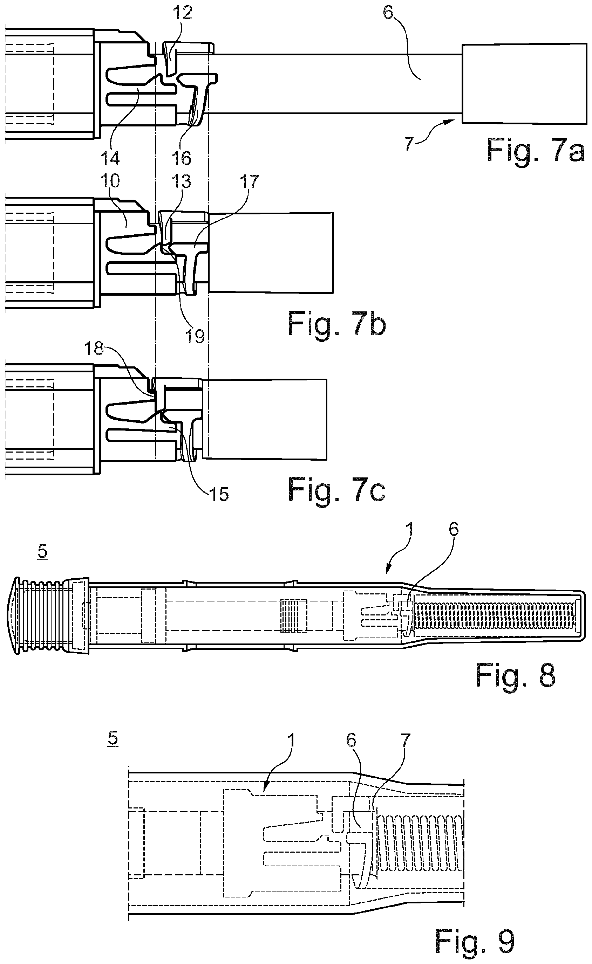

More particular, as also illustrated in **Figs. 7a****-c** the resilient arms 12, 14, 16 are arranged such that moving a substantially planar actuation surface 7, e.g. of an actuation sleeve 6, against the acoustic signal generating device 1 by a first distance presses the first free end 13 of the first resilient arm 12 against the abutment surface 19 of the second resilient arm 14 (s. Fig. 7b), wherein further moving the substantially planar actuation surface 7 against the acoustic signal generating device 1 by an additional second distance in the same direction deflects the third resilient arm 16 such that it disengages the abutment surface 19 from engagement with the first free end 13 (s. Fig. 7c).

Furthermore, particularly from **Fig. 7c** the skilled person understands that the resilient arms 12, 14, 16 are arranged such that they return to the initial not tensioned state (s. Fig. 7a) after being unloaded. As in **Fig. 7a**, the resilient arms 12, 14, 16 do not contact each other when not being loaded. Hence the depicted acoustic signal generating device 1 is only under tension/stress during actuation.

As shown in **Figs. 3 to 6**, the base body 10 has a substantially hollow cylindrical shape, wherein the first resilient arm 12 and the third resilient arm 16 are movable in a direction which is substantially parallel to the longitudinal direction of the base body 10. Further, the first resilient arm 12 and the third resilient arm 16 each comprise a curved portion that extends substantially along the circumferential direction of the substantially hollow cylindrical base body 10. Moreover, the second resilient arm 14 is substantially movable along the circumferential direction of the substantially hollow cylindrical base body 10, wherein the second resilient arm 14 extends substantially parallel to the longitudinal direction of the base body 10.

**Fig. 8** shows a medical injection device 5 comprising the acoustic signal generating device 1 as described above. **Fig. 9** is a detail of Fig. 8. The medical injection device 5 comprises a hollow cylindrical actuation sleeve 6 having an actuation surface 7 for actuating the acoustic signal generating device 1. Said actuation sleeve 6 is arranged slidably relative to the base body 10. The actuation surface 7 is substantially planar.

### List of reference signs

- 1: acoustic signal generating device
- 5: medical injection device
- 6: actuation sleeve
- 7: planar actuation surface
- 10: base body
- 12: first resilient arm
- 13: first free end
- 14: second resilient arm
- 15: second free end
- 16: third resilient arm
- 17: third free end
- 18: impact surface
- 19: abutment surface

## Claims

1. An acoustic signal generating device (1) for a medical injection device (5), the acoustic signal generating device (1) having a base body (10) comprising
a first resilient arm (12) comprising a first free end (13);
an abutment surface (19), and
an impact surface (18),
wherein the first resilient arm (12) is configured to be deflected by a first load being applied on the first resilient arm (12) such that the first free end (13) of the first resilient arm (12) is in compressive engagement with the abutment surface (19), whereby when the first load is increased to a second load the first free end (13) is released from compressive engagement with the abutment surface (19) and the first resilient arm (12) hits the impact surface (18), thereby generating an acoustic signal.

2. The acoustic signal generating device (1) according to the preceding claim, wherein the base body (10) further comprises
a second resilient arm (14), wherein the abutment surface (19) is arranged on the second resilient arm (14), whereby the second resilient arm (14) is configured such that when the first load is increased to the second load the second resilient arm (14) is deflected to an extent that the first free end (13) is released from compressive engagement with the abutment surface (19).

3. The acoustic signal generating device (1) according to the preceding claim, wherein the base body (10) further comprises
a third resilient arm (16) being configured to be deflected such that when the first load is increased to the second load the third resilient arm (16) disengages the abutment surface (19) from compressive engagement with the first free end (13).

4. The acoustic signal generating device (1) according to the preceding claim, wherein the first resilient arm (12), the second resilient arm (14), and the third resilient arm (16) are arranged such that moving a substantially planar actuation surface (7) against the acoustic signal generating device (1) by a first distance presses the first free end (13) against the abutment surface (19), wherein further moving the substantially planar actuation surface (7) against the acoustic signal generating device (1) by an additional second distance deflects the third resilient arm (16) such that it disengages the abutment surface (19) from engagement with the first free end (13).

5. The acoustic signal generating device (1) according to any one of claims 3 or 4, wherein the second resilient arm (14) comprises a second free end (15) and the third resilient arm (16) comprises a third free end (17), wherein the second free end (15) and the third free end (17) are configured to engage with each other when the third resilient arm (16) is deflected.

6. The acoustic signal generating device (1) according to any one of the preceding claims, wherein the base body (10) is integrally formed with the first resilient arm (12), the second resilient arm (14), the third resilient arm (16), the abutment surface (19), and/or the impact surface (18).

7. The acoustic signal generating device (1) according to any one of the preceding claims, wherein the base body (10), the first resilient arm (12), the second resilient arm (14), the third resilient arm (16), the abutment surface (19), and/or the impact surface (18) are injection moulded.

8. The acoustic signal generating device (1) according to any one of the preceding claims, wherein the first resilient arm (12), the second resilient arm (14), and/or the third resilient arm (16) are arranged such that they return to the initial not deflected state after being unloaded.

9. The acoustic signal generating device (1) according to any one of the preceding claims, wherein the first resilient arm (12), the second resilient arm (14), and/or the third resilient arm (16) do not contact each other when not being loaded.

10. The acoustic signal generating device (1) according to any one of the preceding claims, wherein the base body (10) has an elongated hollow shape, wherein the base body (10) optionally has a substantially hollow cylindrical shape.

11. The acoustic signal generating device (1) according to the preceding claim, wherein the first resilient arm (12) and/or the third resilient arm (16) are movable in a direction which is substantially parallel to the longitudinal direction of the base body (10).

12. The acoustic signal generating device (1) according to any one of claims 10 to 11, wherein the first resilient arm (12) and/or the third resilient arm (16) comprise a curved portion that extends substantially along the circumferential direction of the base body (10).

13. The acoustic signal generating device (1) according to any one of claims 10 to 12, wherein the second resilient arm (14) is substantially movable along the circumferential direction of the base body (10), wherein optionally the second resilient arm (14) extends substantially parallel to the longitudinal direction of the base body (10).

14. A medical injection device (5) comprising the acoustic signal generating device (1) according to any one of the preceding claims.

15. The medical injection device (5) according to the preceding claim, wherein the medical injection device (5) comprises the acoustic signal generating device (1) according to any one of claims 10 to 13, wherein the medical injection device (5) further comprises a hollow cylindrical actuation sleeve (6) having an actuation surface (7) for actuating the acoustic signal generating device (1), wherein the actuation sleeve (6) is arranged slidably relative to the base body (10), wherein optionally the actuation surface (7) is substantially planar.
